# EUROPEAN PATENT APPLICATION

(11) **EP 3 957 311 A1**
(43) Date of publication of application: **23.02.2022**
(21) Application number: 20305937.3
(22) Date of filing: 20.08.2020
(51) Int. Cl.: A61K 31/734, A61K 33/04, A61K 31/19, A61K 31/194, A61K 31/197, A61K 33/06, A61K 33/42, A61K 38/00, A61K 45/06, A61P 19/00, A61P 43/00

(54) **ALGINATE HYDROGEL AND NEW COMPOSITION DERIVED THEREFROM FOR THE TREATMENT OF ECTOPIC CALCIFICATIONS**

(71) Applicant: ASSISTANCE PUBLIQUE, HOPITAUX DE PARIS, 75004 Paris (FR)
(72) Inventor: The designation of the inventor has not yet been filed
(74) Representative: Novagraaf Technologies

(57) **Abstract**

The invention concerns an alginate hydrogel or a new composition derived therefrom further comprising another calcium chelator such as sodium thiosulfate and/or calcium crystal solubilizer or inhibitor, and their use in the treatment of ectopic calcifications or disorders comprising ectopic calcifications.

## Description

### Technical field of the invention

The invention concerns an alginate hydrogel or a new composition derived therefrom further comprising another calcium chelator such as sodium thiosulfate and/or calcium crystal solubilizer or inhibitor, and their use in the treatment of ectopic calcifications or disorders comprising ectopic calcifications.

The invention finds an application in the medical field.

In the description below, references in square brackets ([ ]) refer to the list of references at the end of the text.

### State of the art

Ectopic calcifications are common complications of metabolic, inflammatory, traumatic, genetic, degenerative and age-related diseases or occur without an identified cause. They can occur in all tissues affecting in particular cutaneous and subcutaneous tissue, tendons, ligaments, muscles and muscle fascia, cartilage and fibrocartilage, vessels and parenchyma [1]. They consist of either calcium phosphate or calcium pyrophosphate crystals.

In soft tissue calcium phosphate calcifications (called calcinosis), they can be classified into three categories based on the main mechanism of their formation. A distinction is therefore made between dystrophic calcifications that occur in injured tissue without abnormal calcium phosphate balance, metastatic calcifications that occur in healthy tissue under conditions of disrupted calcium phosphate metabolism, and idiopathic calcifications that occur in healthy tissue without identified abnormalities of the calcium phosphate metabolism. The causes of dystrophic calcifications are multiple. These calcifications can occur after physical trauma (e.g. crusching of limbs, war wounds) of varying degrees of severity, hematoma, infection (e.g. abscess or cellulitis), tissue necrosis, irradiation, physical or chemical burns, tumor lesions, degenerative lesions or inflammatory lesions. In connective tissue diseases such as scleroderma, dermatomyositis or lupus, calcinosis are a major complication. They occur in 10-50% of scleroderma and 30-70% of dermatomyositis [2,3]. Metastatic calcinosis occur during end-stage renal failure and in dialysis patients, in conditions with chronic increase of calcium and/or phosphorus such as vitamin D overload, sarcoidosis, or genetic diseases with mutations of *FGF23, KLOTHO* or *GALNT3* genes. These mutations are responsible for hyperphosphatemic familial tumoral calcinosis (HFTC) which causes voluminous and disabling calcifications with sometimes fatal complications [4]. They vary in size and are responsible for pain, recurrent inflammatory reactions, skin fistulizations with superinfections, and local compressions. Tendon calcifications are frequent, most often affecting the shoulders but all sites can be affected. In the shoulder the prevalence varies from 2.7% to more than 30%. They are most often bilateral. They also generate chronic pain and inflammatory flare-ups [5,6].

Calcium pyrophosphate deposits are favoured by ageing and several metabolic diseases (e.g. hyperparathyroidism, haemochromatosis, hypomagnesemia, hypophosphatasia, copper overload, ochronosis) and genetic diseases. They affect the articular cartilage, intervertebral discs, fibrocartilages, ligaments and tendons. They are also responsible for acute and chronic pain and recurrent inflammatory reactions [7,8].

In brief, calcium crystal formation is secondary to imbalance between pro- and anti-mineralization factors, extracellular matrix and cell alterations and disturbances of calcium, phosphate and pyrophosphate concentrations [9,10]. Inorganic pyrophosphate (PPi) is a major inhibitor of calcium phosphate and hydroxyapatite crystal formation by preventing crystal nucleation and crystal growth [11-13]. Its deficiency favors ectopic calcification as observed in several diseases such as Generalized Artery Calcification in Infancy (GACI), pseudoxanthoma elasticum (PXE), vascular calcifications in chronic kidney disease (CKD) [14-16]. In contrast, increase concentration of PPi can lead to calcium pyrophosphate crystal formation [7,8]. Extracellular PPi concentration is composed of intracellular export through ANK transporter and its extracellular formation through nucleotides hydrolysis by ectonucleotide pyrophosphatases/phosphodiesterases (ENPP). Extracellular ATP is hydrolyzed by ENPP1 to AMP and PPi. PPi is hydrolyzed by tissue non-specific alkaline phosphatase (TNAP) into 2 inorganic phosphate (Pi) while AMP is metabolized into Pi and adenosine by CD73. Adenosine is an inhibitor of TNAP [13,14,17,18]. Decrease of TNAP activity secondary to genetic mutation or alteration of its co-factors such as hypomagnesemia lead to PPi accumulation and calcium pyrophosphate crystal formation [18]. PPi deficiency due to *ENPP1* mutation or increase TNAP activity as observed in *NT5E* mutation favors calcium phosphate crystal formation. In pre-clinical studies, enriched-PPi diet prevents and reverses ectopic calcifications [19,20].

Calcium phosphate and calcium pyrophosphate crystals are associated with osteoarthritis. In the advanced stage, all osteoarthritic cartilages are calcified. Calcifications of cartilage aggravate osteoarthritis disease [21-23].

Arterial calcifications are made up of calcium phosphate crystals, accompany atherosclerosis and several metabolic diseases (type 2 diabetes, chronic kidney disease and genetic diseases), and are responsible for arterial stenosis and occlusion [24].

There is currently no effective treatment to dissolve calcium crystals already deposited in tissues. Several treatments have been tested in calcinosis with inconsistent results: bisphosphonates such as etidronate and pamidronate, high dose of calcium channel inhibitors such as diltiazem, probenecid, shock waves, anti-TNF, anti-CD20 and sodium thiosulfate (25].

Among these proposed treatments, sodium thiosulfate appears promising with a reported efficacy in calcifications of arterioles or calciphylaxis in dialysis patients [26] and in some cases of calcinosis complicating dermatomyositis, scleroderma or during hyperphosphatemic familial tumoral calcinosis (HFTC) [27,28].

So there is still a need for an effective treatment of ectopic calcifications whatever their origins.

Alginate is a known calcium-binding exopolysaccharide composed of mannuronic and guluronic acid, obtained from brown algae. Alginate can be used as additives (e.g. thickeners, gelling agents, emulsifiers and stabilizers) for a wide range of industrial products from food and cosmetic products to paint and printing inks. Alginate can also be used in medicine to encapsulate drugs or fragile biological substances, or to manufacture gastric bandages and dressings for superinfected or non-superinfected skin lesions with or without a risk of bleeding and in pressure ulcers.

### Description of the invention

Although the calcium chelating properties of alginates have never been used therapeutically in ectopic calcifications, the Inventors have shown that an advantageously formulated alginate hydrogel injected as close as possible to the therapeutic target can reduce calcification. Alginate hydrogel can act as a chelating agent by ion exchange, with calcium forming the pathological crystals and thus optimise its effectiveness to treat ectopic calcifications. Alternatively, alginate hydrogel can modulate the phenotype of giant cells that surrounded calcium deposits toward a pro-resorbing phenotype. Such alginate hydrogel may be combined with other calcium chelators such as sodium thiosulfate, EDTA, acetic acid (or other acids) or inhibitors of calcium crystal deposition. In particular, such hydrogel has been formulated to be able to carry sodium thiosulfate.

An object of the present invention is therefore a composition of alginate d'hydrogel further comprising at least one other calcium chelator and/or calcium crystal solubilizer or inhibitor.

According to a particular embodiment of the composition of the present invention, the at least one other calcium chelator is chosen from the group consisting of sodium thiosulfate, EDTA, and acetic acid. Preferably, it is sodium thiosulfate.

According to a particular embodiment of the composition of the present invention, the at least one calcium crystal solubilizer is chosen from the group consisting of EDTA, EGTA, citrate, hydroxycitrate, citric acid or other acids [29-32].

According to a particular embodiment of the composition of the present invention, the at least one calcium crystal inhibitor is chosen from the group consisting of pyrophosphate, magnesium, fetuin A, ENPP1 or osteopontin [1,11,13,33,34].

Another object of the present invention is also an alginate hydrogel or a composition of the present invention, for use in the prevention or treatment of ectopic calcifications or disorders comprising ectopic calcifications.

According to a particular embodiment of the present invention, the disorders comprising ectopic calcifications are chosen from the group consisting of metabolic, inflammatory, traumatic, genetic, degenerative and age-related diseases. For example, the disorders are chosen from the group consisting of osteoarthrosis, physical trauma, hematoma, infection, tissue necrosis, irradiation, physical or chemical burns, tumor lesions, degenerative or inflammatory lesions, scleroderma, dermatomyositis, lupus, calcinosis, sarcoidosis, familial hyperphosphatemic or normophosphatemic tumoral calcinosis, hyperparathyroidism, haemochromatosis, hypomagnesemia, hypophosphatasia, vitamin D or copper overload, ochronosis, arthritis, type 2 diabetes, chronic kidney disease.

### Brief description of the figures

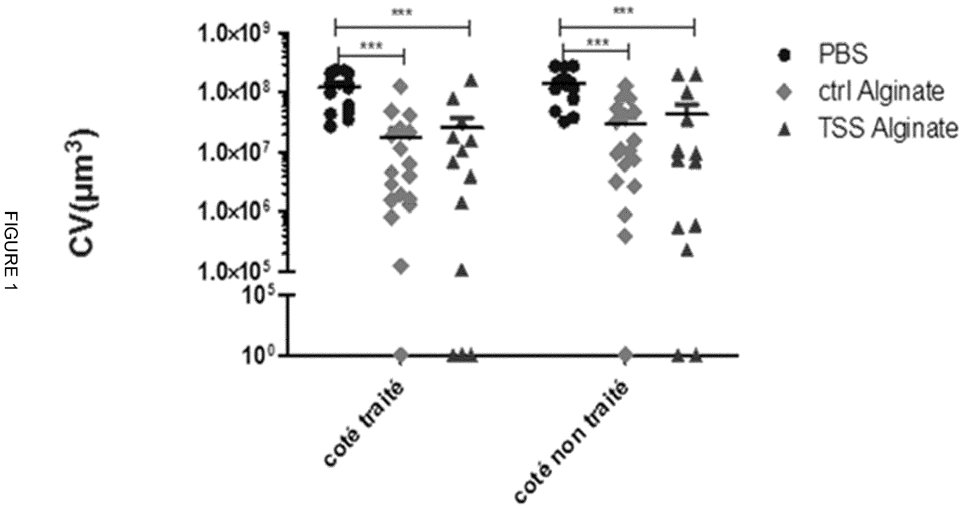

Figure 1 represents the evolution of the volume (µm³) of cryo-induced muscle calcification in mice following two local injections of saline (● ; n=14) or alginate (◆ ; n=18), or alginate associated with sodium thiosulfate (▲ ; n=14) **p<0.001.

### EXAMPLES

### EXAMPLE 1: TREATMENT OF ECTOPIC CALCIFICATIONS IN A MOUSE MODEL USING AN ALGINATE HYDROGEL ALONE OR ASSOCIATED WITH SODIUM THIOSULFATE

### Quadriceps calcifications were induced by cryoinjury in right and left sides:

Mice (mouse model of cryo-induced muscle calcification.) were anaesthetized with ketamine/xylazine. After cutaneous incision, quadriceps injuries were induced by 15-second freeze-thaw procedure using a liquid nitrogen-cooled surgical forceps of 6 mm wide. Mice were killed at different time points (from baseline to day 28 after cryoinjury) and quadriceps were harvested and fixed in 4% PFA overnight. Calcifications were quantified by micro-CT using. In this model, calcifications appeared at D1 and plateaued at D7 to D28.

### Compositions of alginate hydrogel used:

Alginate hydrogel sample (Protanal LF 10/60 1.2% m/V)
- 1.2 mg of sodium alginate were dissolved in 100 mL of sterile water under stirring (550 rpm). After dissolution, solution was stored at 4°C overnight. Then, alginate solution was filtered through Sartolab P20 filter under sterile hood. The solution was stored at 4°C used within 4 weeks.
Sodium thiosulfate 10% m/V in Protanal LF 10/60 1.2% m/V :
- 2.5 mg of sodium thiosulfate were dissolved in 25 ml of protanal LF 10/60 1.2% under stirring and then filtered through Sartolab P20 filter under sterile hood. Solution was stored at 4°C and used within 4 weeks.
Sodium hyaluronate 0.14% m/V in Protanal LF 10/60 1.20% m/V:
- 28 µg of sodium hyaluronate were dissolved in 20 ml of sterile protanal LF 10/60 1.2% under stirring. After dissolution, solution was stored at 4°C until used.

### Protocol:

Using this cryo-induced muscle calcification, the effect of alginate hydrogel Protanal LF 10/60 1.2% m/v alone or associated with sodium thiosulfate 10% m/v, was assessed.

At D7 after cryoinjury, mice received under sedation local injections with a 25G needle into right quadriceps of 50 µL of either saline buffer (PBS) (n=14) or alginate hydrogel 1.2% (n=18) or alginate TSS (n=14). Left quadriceps were not treated. Same treatment was repeated at D10. After these two injections spaced 3 days apart, at D14, left and right quadriceps were harvested and calcification volumes were quantified by microscanning muscle samples.

The results presented in Figure 1 showed that two local injections of alginate or alginate TSS spaced three days apart resulted in a substantial and significant decrease in muscle calcifications. Interestingly, calcification reduction in treated sides and untreated contralateral sides was observed, suggesting a systemic effect.

### List of references

1. Reznikov N, Hoac B, Buss DJ, Addison WN, Barros NMT, McKee MD. Biological stenciling of mineralization in the skeleton: Local enzymatic removal of inhibitors in the extracellular matrix. Bone. 2020 Sep 1;138:115447.
2. Cruz-Domínguez MP, García-Collinot G, Saavedra MA, Medina G, Carranza-Muleiro RA, Vera-Lastra OL, et al. Clinical, biochemical, and radiological characterization of the calcinosis in a cohort of Mexican patients with systemic sclerosis. Clin Rheumatol. 2017 Jan 1;36(1):111-7.
3. Valenzuela A, Song P, Chung L. Calcinosis in scleroderma. Curr Opin Rheumatol. 2018;30(6):554-61.
4. Boyce AM, Lee AE, Roszko KL, Gafni RI. Hyperphosphatemic Tumoral Calcinosis: Pathogenesis, Clinical Presentation, and Challenges in Management. Front Endocrinol [Internet]. 2020 [cited 2020 Jun 28];11. Available from: https://www.frontiersin.org/articles/10.3389/fendo.2020.00293/full
5. Louwerens JKG, Sierevelt IN, van Hove RP, van den Bekerom MPJ, van Noort A. Prevalence of calcific deposits within the rotator cuff tendons in adults with and without subacromial pain syndrome: clinical and radiologic analysis of 1219 patients. J Shoulder Elbow Surg. 2015 Oct;24(10):1588-93.
6. Witte PB de, Adrichem RA van, Selten JW, Nagels J, Reijnierse M, Nelissen RGHH. Radiological and clinical predictors of long-term outcome in rotator cuff calcific tendinitis. Eur Radiol. 2016 Mar 5,1-11.
7. Rosenthal AK, Ryan LM. Calcium Pyrophosphate Deposition Disease. N Engl J Med. 2016 Jun 30;374(26):2575-84.
8. Abhishek A, Doherty M. Pathophysiology of articular chondrocalcinosis-role of ANKH. Nat Rev Rheumatol. 2011 Feb;7(2):96-104.
9. Li Q, Arányi T, Váradi A, Terry SF, Uitto J. Research Progress in Pseudoxanthoma Elasticum and Related Ectopic Mineralization Disorders. J Invest Dermatol. 2016 Mar;136(3):550-6.
10. Back M, Aranyi T, Cancela ML, Carracedo M, Conceição N, Leftheriotis G, et al. Endogenous Calcification Inhibitors in the Prevention of Vascular Calcification: A Consensus Statement From the COST Action EuroSoftCalcNet. Front Cardiovasc Med [Internet]. 2019 [cited 2020 Jul 5];5. Available from: https://www.frontiersin.org/articles/10.3389/fcvm.2018.00196/full
11. Fleisch H, Schibler D, Maerki J, Frossard I. Inhibition of aortic calcification by means of pyrophosphate and polyphosphates. Nature. 1965 Sep 18;207(5003):1300-1.
12. Russell RGG, Bisaz S, Donath A, Morgan DB, Fleisch H. Inorganic pyrophosphate in plasma in normal persons and in patients with hypophosphatasia, osteogenesis imperfecta, and other disorders of bone. J Clin Invest. 1971 May 1;50(5):961-9.
13. Orriss IR. Extracellular pyrophosphate: The body's "water softener." Bone. 2020 May 1;134:115243.
14. Rutsch F, Ruf N, Vaingankar S, Toliat MR, Suk A, Höhne W, et al. Mutations in ENPP1 are associated with "idiopathic" infantile arterial calcification. Nat Genet. 2003 Aug;34(4):379-81.
15. Villa-Bellosta R, O'Neill WC. Pyrophosphate deficiency in vascular calcification. Kidney Int. 2018 Jun 1;93(6):1293-7.
16. Sánchez-Tévar AM, García-Fernández M, Murcia-Casas B, Rioja-Villodres J, Carrillo JL, Camacho M, et al. Plasma inorganic pyrophosphate and alkaline phosphatase in patients with pseudoxanthoma elasticum. Ann Transl Med [Internet]. 2019 Dec [cited 2020 Jun 22];7(24). Available from: https://www.ncbi.nlm.nih.gov/pmc/articles/PMC6989879/
17. St. Hilaire C, Ziegler SG, Markello TC, Brusco A, Groden C, Gill F, et al. NT5E Mutations and Arterial Calcifications. N Engl J Med. 2011 Feb 3;364(5):432-42.
18. Whyte MP. Hypophosphatasia - aetiology, nosology, pathogenesis, diagnosis and treatment. Nat Rev Endocrinol. 2016 Apr;12(4):233-46.
19. Pomozi V, Brampton C, van de Wetering K, Zoll J, Calio B, Pham K, et al. Pyrophosphate Supplementation Prevents Chronic and Acute Calcification in ABCC6-Deficient Mice. Am J Pathol. 2017 Jun;187(6):1258-72.
20. Dedinszki D, Szeri F, Kozak E, Pomozi V, Tőkési N, Mezei TR, et al. Oral administration of pyrophosphate inhibits connective tissue calcification. EMBO Mol Med. 2017 Nov 1;9(11):1463-70.
21. Nguyen C, Bazin D, Daudon M, Chatron-Colliet A, Hannouche D, Bianchi A, et al. Revisiting spatial distribution and biochemical composition of calcium-containing crystals in human osteoarthritic articular cartilage. Arthritis Res Ther. 2013;15(5):R103.
22. Fuerst M, Bertrand J, Lammers L, Dreier R, Echtermeyer F, Nitschke Y, et al. Calcification of articular cartilage in human osteoarthritis. Arthritis Rheum. 2009 Sep 1;60(9):2694-703.
23. Hawellek T, Hubert J, Hischke S, Vettorazzi E, Wegscheider K, Bertrand J, et al. Articular cartilage calcification of the humeral head is highly prevalent and associated with osteoarthritis in the general population. J Orthop Res. 2016 Apr 1;n/a-n/a.
24. Daniela Q, Federica B, Lofaro FD. Chapter Eight - The biology of vascular calcification. In: Galluzzi L, editor. International Review of Cell and Molecular Biology [Internet]. Academic Press; 2020 [cited 2020 Jun 2]. p. 261-353. Available from: http://www.sciencedirect.com/science/article/pii/S193764482030023X
25. Traineau H, Aggarwal R, Monfort J-B, Senet P, Oddis CV, Chizzolini C, et al. Treatment of calcinosis cutis in systemic sclerosis and dermatomyositis: A review of the literature. J Am Acad Dermatol. 2020 Feb 1;82(2):317-25.
26. Peng T, Zhuo L, Wang Y, Jun M, Li G, Wang L, et al. A systematic review of sodium thiosulfate in treating calciphylaxis in chronic kidney disease patients. Nephrology. :n/a-n/a.
27. Jost J, Bahans C, Courbebaisse M, Tran T-A, Linglart A, Benistan K, et al. Topical Sodium thiosulfate: a treatment for calcifications in hyperphosphatemic familial tumoral calcinosis? J Clin Endocrinol Metab. 2016 May 10;jc.2016-1087.
28. Goossens J, Courbebaisse M, Caudron E, Bahans C, Vacquerie V, Melchior J, et al. Efficacy of intralesional sodium thiosulfate injections for disabling tumoral calcinosis: Two cases. Semin Arthritis Rheum. 2017 Dec;47(3):451-5.
29. Chutipongtanate S, Chaiyarit S, Thongboonkerd V. Citrate, not phosphate, can dissolve calcium oxalate monohydrate crystals and detach these crystals from renal tubular cells. Eur J Pharmacol. 2012 Aug 15;689(1):219-25.
30. Chung J, Granja I, Taylor MG, Mpourmpakis G, Asplin JR, Rimer JD. Molecular modifiers reveal a mechanism of pathological crystal growth inhibition. Nature. 2016 Aug;536(7617):446-50.
31. Wang L, Nancollas GH. Calcium Orthophosphates: Crystallization and Dissolution. Chem Rev. 2008 Nov;108(11):4628-69.
32. Dorozhkin SV. Dissolution mechanism of calcium apatites in acids: A review of literature. World J Methodol. 2012 Feb 26,2(1):1-17.
33. Steitz SA, Speer MY, McKee MD, Liaw L, Almeida M, Yang H, et al. Osteopontin inhibits mineral deposition and promotes regression of ectopic calcification. Am J Pathol. 2002 Dec;161(6):2035-46.
34. Babler A, Schmitz C, Buescher A, Herrmann M, Gremse F, Gorgels T, et al. Microvasculopathy and soft tissue calcification in mice are governed by fetuin-A, magnesium and pyrophosphate. PloS One. 2020;15(2):e0228938.

## Claims

1. Composition of alginate hydrogel further comprising at least one other calcium chelator and/or calcium crystal solubilizer or inhibitor.

2. Composition according to claim 1, wherein the at least one other chelator is chosen from the group consisting of sodium thiosulfate, EDTA, and acetic acid.

3. Composition according to claim 1, wherein the at least one calcium crystal solubilizer is chosen from the group consisting of EGTA, EDTA, citrate, hydroxycitrate.

4. Composition according to claim 1, wherein the at least one calcium crystal inhibitor is chosen from the group consisting of pyrophosphate, magnesium, fetuin A, ENPP1, osteopontin.

5. Alginate hydrogel or composition as defined in anyone of claims 1 to 4, for use in the prevention or treatment of ectopic calcifications or disorders comprising ectopic calcifications.

6. Alginate hydrogel or composition as defined in anyone of claims 1 to 4 for use according to claim 5, wherein the disorders comprising ectopic calcifications are chosen from the group consisting of metabolic, inflammatory, traumatic, genetic, degenerative and age-related diseases.

7. Alginate hydrogel or composition as defined in anyone of claims 1 to 4 for use according to claim 6, wherein disorders are chosen from the group consisting of osteoarthrosis, physical trauma, hematoma, infection, tissue necrosis, irradiation, physical or chemical burns, tumor lesions, degenerative or inflammatory lesions, scleroderma, dermatomyositis, lupus, calcinosis, sarcoidosis, familial hyperphosphatemic or normophosphatemic tumoral calcinosis, hyperparathyroidism, haemochromatosis, hypomagnesemia, hypophosphatasia, vitamin D or copper overload, ochronosis, arthritis, type 2 diabetes, chronic kidney disease.
